# EUROPEAN PATENT APPLICATION

(11) **EP 3 202 396 A1**
(43) Date of publication of application: **09.08.2017**
(21) Application number: 17154019.8
(22) Date of filing: 31.01.2017
(51) Int. Cl.: A61K 31/131, A61K 31/15

(54) **PHARMACEUTICAL COMPOSITION FOR USE IN ANTICOAGULATION**

(30) Priority: 03.02.2016 US 201615014373
(71) Applicant: Wang, Soo-Ray, Taichung 40201 (TW)
(72) Inventor: Wang, Soo-Ray, Taichung 40201 (TW)
(74) Representative: Fuchs Patentanwälte Partnerschaft mbB

(57) **Abstract**

The invention provides a pharmaceutical composition for use in anticoagulation, wherein the pharmaceutical composition including formula (I) or its isomers as its parent form, and its salt, ester or solvate. The invention also provides a pharmaceutical composition for use in anticoagulation, wherein the pharmaceutical composition including formula (I) or its isomers as its parent form, and its salt, ester or solvate, wherein a dose of the pharmaceutical composition for use in anticoagulation is in an amount of about 5x10⁻⁵ mL/kg or more of body weight of the subject.

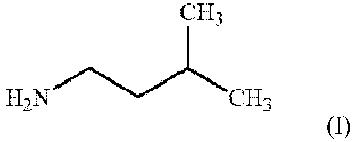

## Description

### CROSS REFERENCE TO RELATED APPILCATIONS

The present application is based on, and claims priority from, pending U.S. patent application Ser. No. 15/014,373, filed on February 3, 2016 and entitled "Method for anticoagulation", the disclosure of which is hereby incorporated by reference herein in its entirety.

### BACKGROUND OF THE INVENTION

### Field of the Invention

The present invention relates to a pharmaceutical composition for use in anticoagulation.

### Description of the Related Art

Coagulation (also known as clotting) is the process by which blood changes from a liquid to a gel. It potentially results in hemostasis, the cessation of blood loss from a damaged vessel, followed by repair. The mechanism of coagulation involves activation, adhesion, and aggregation of platelets along with deposition and maturation of fibrin. Coagulation is highly conserved throughout biology. In all mammals, coagulation involves both a cellular (platelet) and protein (coagulation factors) component. The system in humans has been the most extensively researched and is the best understood.

Disorders of coagulation are disease states which can result in bleeding (hemorrhage or bruising) or obstructive clotting (thrombosis). For example, thrombosis includes venous thrombosis and arterial thrombosis. Specifically, venous thrombosis includes such as deep vein thrombosis, portal vein thrombosis, renal vein thrombosis, jugular vein thrombosis, Budd-Chiari syndrome, Paget-Schroetter disease, cerebral venous sinus thrombosis, cavernous sinus thrombosis, and arterial thrombosis includes such as stroke and myocardial infarction.

Currently, most commonly used anticoagulative agents in clinic include heparin and coumarin. Heparin activates antithrombin III, which in turn inhibits coagulate factor XII a, XIa, IXa, Xa, and IIa. Coumarin is an oral anticoagulative agent, which depletes vitamin K.

On the other hand, septic shock is a life-threatening complication of bacterial infection. Serious sepsis and septic shock are among the main reasons causing death in hospital of infected patients. So-called sepsis refers to a whole-body inflammatory state (called a systemic inflammatory response syndrome, or SIRS) and the presence of an obvious infection. Septic shock is frequently complicated by the syndrome of disseminated intravascular coagulation (DIC) due to a massive activation of the coagulation system. While the human body itself also provides treatment for septic shock. Many biological molecules are involved in anticoagulation pathway, such as activated protein C.

The main symptoms of septic shock include fever, chills, high respiratory rate and confusion/coma etc., which also include elevated or lowered body temperature (over 38.3 °C or under 36 °C), a heart rate over 90 beats per minute, a respiratory rate over 20 breaths per minute, and a white blood cell count over 12,000/cu.mm, or less than 4,000/cu.mm.

An even worse situation of sepsis may result in hypotension, hypoperfusion, and multiple organ failure, which may include reduced urine flow (renal failure), abnormal liver function, and jaundice. This condition of vital organ dysfunction due to insufficient blood flow is called septic shock.

In the case of bacterial infection, activation of the host's immune cells occurs with the release of cytokine and non-cytokine mediators, the most notorious of which are tumor necrosis factor-alpha (TNF-*α*), interleukin 1 (IL-1) and interleukin 6 (IL-6). These factors are implicated in the diffuse activation of a systematic inflammatory response. As a result, mediators with vasodilatory and endotoxic properties are released systematically, including prostaglandins, thromboxane A2 and nitric acid. This results in vasodilatation and endothelial damage, which lead to hypotension and capillary leak. In addition, cytokine activates the coagulation pathway, resulting in capillary microthrombi and end-organ ischemia.

There has been research on several treatments for septic shock, comprising steroids, recombinant APC (activated protein C), and many chemical compounds.

Protein C is a major anticoagulation component which functions like a vitamin K-dependent serine protease enzyme. Protein S functions as a cofactor to protein C in the inactivation of coagulation factors V and VIII. Protein C is activated in a sequence that starts with protein C and thrombin binding to a cell surface protein thrombomodulin. The activated protein C, along with protein S and a phospholipid as cofactors, degrades coagulation factor V and VIII, thus inhibiting coagulation.

According to Bernard G R et al., 2001, recombinant human activated protein C has anti-thrombotic, anti-inflammatory and profibrinolytic properties. Based on earlier reports, it is known that APC can protect animals and humans from septic shock. However, the effects of APC remain to be discovered. Nacira S had demonstrated the protective effect of APC, prevented the reduction of blood pressure induced by LPS, and improved both vascular hyperreactivity and myocardial performance in rats. This effect was associated with decreased up-regulation of NF-kappaB, iNOS and MMP-9 (Nacira S et al., Crit. Care Med. 2009 January; 37(1):246-55). Although it was effective, a large randomized clinical trial demonstrated only a 6.1% absolute decrease in mortality (from 30.8 to 24.7%) among patients with severe sepsis, while efficacy along with side effect exists (Bernard G. R. et al., N Engl J Med 2001, 8; 344(10):699-709). Moreover, on October 25, 2011, Eli Lilly & Co. withdrew Xigris (trade name of APC) from the market after a major study showed no efficacy for the treatment of sepsis ("Xigris (drotrecogin alfa (activated)) to be withdrawn due to lack of efficacy". Press release. London, UK: European Medicines Agency. 25 October 2011. Archived from the original on 26 October 2011. Retrieved 26 October 2011).

Besides, US. Pat. No. 4,388,318 disclosed a method of treating endotoxin shock with a pyrimido-pyrimidine derivative. Since *E. coli* endotoxin may exert its hypotensive effect by activating automatic blood pressure regulatory circuit in central nervous system, the administering of a pyrimido-pyrimidine derivative will have hypertensive effect acting on the medullary cardiovascular regulatory system.

US. Pat. No. 6,011,066 disclosed an amine compound of chemical formula C₂H₂R1R2R3NH₂ for treating septic shock and a chemical structure thereof, wherein R1 and R2 are chosen from hydrogen, hydrocarbon, carboxyl group, amine group or alkyl group containing 1-8 carbons, and R3 is chosen from hydrogen, hydrocarbon, carboxyl group, phenyl group and its substitution, acrylamine, alkylamine containing 3-8 carbons, alkylamino-carboxylic acid or an effective amount of their salt, ester or solvate. The amine compound was administered to a subject orally or parenterally.

US. Pat. No. 8,557,873 disclosed a method for treating septic shock or endotoxemia with isoamylamine (IA). However, the mechanism remains unknown.

As mentioned above, a wide variety of diseases are related with coagulation and a massive activation of the coagulation system caused disseminated intravascular coagulation (DIC) affects septic shock. Also, coagulopathy has been attributed as partly a cause of cardiovascular disease.

Therefore, a method of treatment having effective bioactivity for inhibiting coagulation is demanded.

### BRIEF SUMMARY OF THE INVENTION

An embodiment of the present invention provides a pharmaceutical composition for use in anticoagulation, wherein the pharmaceutical composition comprises formula (I): 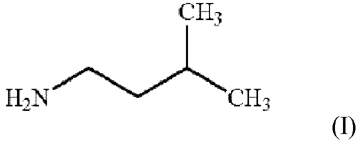 or its isomers as its parent form, and its salt, ester or solvate.

Another embodiment of the present invention provides a pharmaceutical composition for use in anticoagulation, wherein the pharmaceutical composition comprises formula (I): 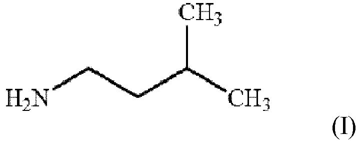 or its isomers as its parent form, and its salt, ester or solvate, wherein a dose of the pharmaceutical composition for use in anticoagulation is in the amount of about 5x10⁻⁵ mL/kg or more of the body weight of the subject.

A detailed description is given in the following embodiments with reference to the accompanying drawings.

### BRIEF DESCRIPTION OF THE DRAWINGS

The present invention can be more fully understood by reading the subsequent detailed description and examples with references made to the accompanying drawings, wherein:

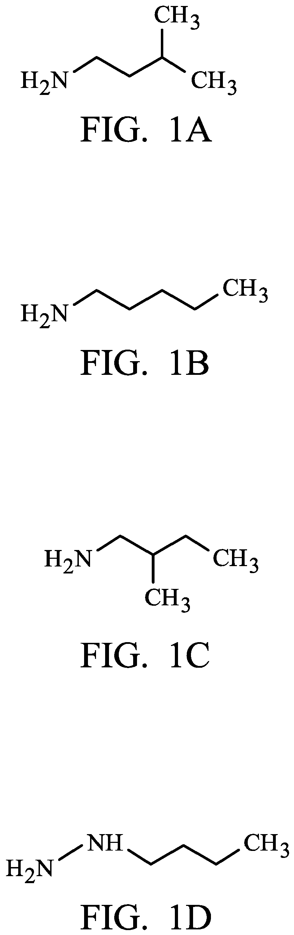

Fig. 1A illustrates the chemical formula of the pharmaceutical composition of the present invention;
Fig. 1B-1D illustrate the chemical formulas of the isomers of the pharmaceutical composition of the present invention;
Fig. 2A shows the active partial thromboplastin time (aPTT) of the isoamylamine (IA)-treated mice with 1 mL of 1000 ppm IA under different treatment times according to some embodiments of the present invention;
Fig. 2B shows the active partial thromboplastin time (aPTT) of the isoamylamine (IA)-treated mice with different dosages for 3hr according to some embodiments of the present invention;
Fig. 3A shows the prothrombin time (PT) of the isoamylamine (IA)-treated mice with 1 mL of 1000 ppm IA under different treatment times according to some embodiments of the present invention; and
Fig. 3B shows the prothrombin time (PT) of the isoamylamine (IA)-treated mice with different dosages for 3hr according to some embodiments of the present invention.

### DETAILED DESCRIPTION OF THE INVENTION

The following description is of the best-contemplated mode of carrying out the invention. This description is made for the purpose of illustrating the general principles of the invention and should not be taken in a limiting sense. The scope of the invention is best determined by reference to the appended claims.

Moderate alcohol consumption reduces mortality from all causes of cardiovascular events through endothelium protection. In particular, red wine drinking contributes to a beneficial effect of hemostasis. IA, a synonym of 3-methyl-1-butanamine or isopentylamine, is an organic chemical compound of colorless liquid and is also one of the components of red wine and grape juice. In accordance with J of agriculture & food chemistry, 2011, 59:8742-53*,* the content of IA in port wine is about 0.4 ppm, and the content of IA in grape juice is about 1.2 ppm. Therefore, IA is edible. The present application shows the IA effects on murine coagulation, which is a cause of cardiovascular events.

An embodiment of the present invention provides a pharmaceutical composition for use in anticoagulation, wherein the pharmaceutical composition including formula (I) or its isomers as its parent form, and its salt, ester or solvate

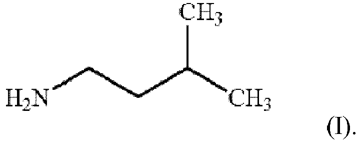

In other embodiments of the present invention, a pharmaceutical composition for use in anticoagulation may include the isomers of the pharmaceutical composition formula (I), such as formulas (II), (III) or (IV) and their salt, ester or solvate

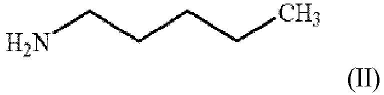

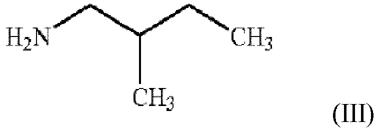

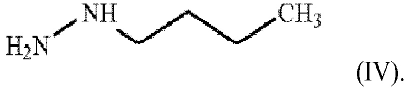

In one embodiment of the present invention, the use of the pharmaceutical composition in anticoagulation may include administering to a subject in need a therapeutically effective amount of pharmaceutical composition including formula (I) or its isomers as its parent form, and its salt, ester or solvate. In another embodiment of the present invention, the use of the pharmaceutical composition in anticoagulation may include administering to a subject in need a therapeutically effective amount of pharmaceutical composition including the isomers of the pharmaceutical composition formula (I), such as formulas (II), (III) or (IV) and their salt, ester or solvate.

In the present invention, a therapeutically effective pharmaceutical composition represents what amount is sufficient to reduce, inhibit, or prevent blood coagulation in an individual, wherein the blood coagulation may be induced by endotoxins, bacteremia, or the like. In one embodiment, a therapeutically effective pharmaceutical composition may be an amount of 5x10⁻⁵mL/kg or more. In one embodiment, a therapeutically effective pharmaceutical composition may be an amount of 5x10⁻⁵ to 0.05 mL/kg, for example, 5x10⁻⁴mL/kg or 5x10⁻³mL/kg, depending on treatment time and the affected pathway (intrinsic or extrinsic).

In one embodiment, the pharmaceutical composition may further include a pharmaceutically acceptable carrier such as phosphate buffer saline or sterile water.

According to one embodiment of the present invention, the pharmaceutical composition may be administered parenterally, for example, in sterile liquid dosage form and intraperitoneally or intravascularly injected into the subject. The pharmaceutical composition may also be administered orally, for example in sterile liquid dosage forms such as syrup and suspension, or in solid dosage forms such as capsules, tables and powder.

In one embodiment, the subject may be a mammal. In another embodiment, the subject may be a mouse. In another embodiment, the subject may be a human.

The examples described below show the effects of IA on coagulation under different dosages and treatment times. It should be noted that the following examples are presented herein to describe the best mode of the present invention, and they are not intended to limit the present invention.

### Materials and Methods

### Isoamylamine (IA)

Isopentylamine (Isoamylamine; M-820716) 250 mL was purchased from Merck, Germany. It was diluted with normal saline to various concentrations for intraperitoneal injection.

### aPTT and PT reagents

aPTT (active partial thromboplastin time) and PT (prothrombin time) reagent were purchased from Dade Behring company, Siemens Healthcare Diagnostics Products GmbH, Marburg, Germany..

### Animals

The 5- to 6-week old male C57BL/6JNarl mice having body weights around 20g were purchased from National Laboratory Animal Center, Taipei, Taiwan.

### Treatments

Coagulation system in blood is composed of intrinsic and extrinsic pathways. The intrinsic pathway is assayed by activated thromboplastin time (aPTT) and the extrinsic pathway is assayed by prothrombin time (PT). Following intraperitoneal injection of various dose of IA, plasma was obtained at different periods of time and was assayed for aPTT and PT. aPTT and PT were assayed in a Stago ST4 coagulation analyzer (ST art 4, Diagnostica Stago, France).

### Preparation of mouse plasma

By carbon dioxide anesthesia of mice, blood was obtained by cardiac puncture using 3.2% sodium citrate mixing with blood at a ratio of 1:9. The blood was centrifuged at 2000g/rpm for 10 minutes to get the mouse plasma, which was assayed immediately.

### Assay of activated partial thromboplastin time (aPTT)

aPTT was assayed in 4-fold dilution of aPTT reagent using kit-provided buffer solution as diluent. In aPTT assay, 0.1mL of plasma was kept warm for 1 min at 37°C in a barrette of 4 cuvettes in Stago ST4 coagulation analyzer, then 0.1mL of 4 fold-diluted aPTT reagent and one steel ball were added and waited for 180 seconds. Finally, 0.1 mL of 25 mM CaCl₂ solution was added for aPTT detection.

### Assay of prothrombin time (PT)

PT was assayed in 4-fold dilution of PT reagent, composed of four parts including one part of PT reagent, one part of 25 mM CaCl₂ solution, and two parts of sterile water. In PT assay, 0.1mL of plasma was put into each of 4 barrette cuvette, each of which had one steel ball, and was kept at 37°C for 1 min in Stago ST4 coagulation analyzer. Then, 0.2 mL of the diluted PT reagent was added for PT detection.

### Example 1: Time course study (aPTT)

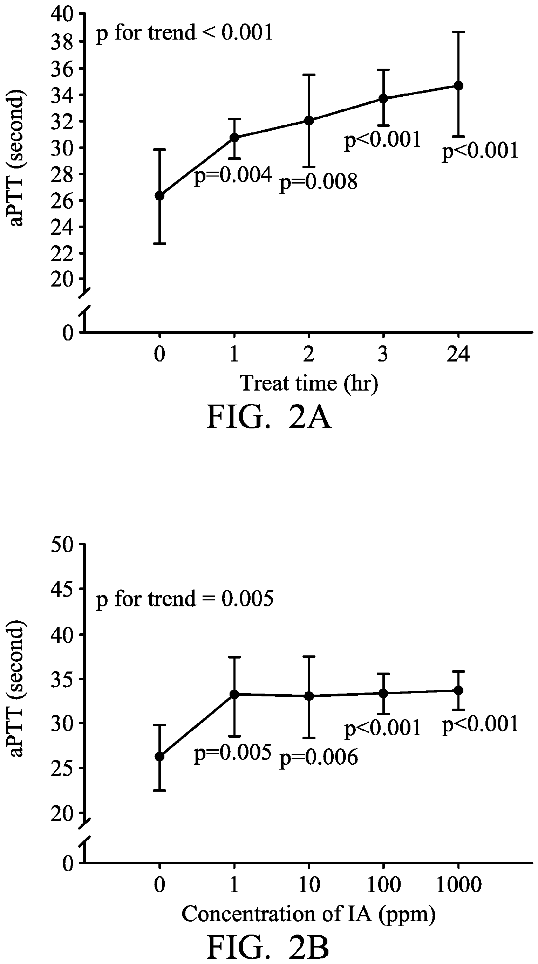

To investigate the anticoagulation effect in the intrinsic pathway of coagulation system in the IA-treated mice, the aPTT was measured at different treatment times. Mouse (n=6) was intraperitoneally injected with 1 mL of 1000 ppm (0.05 mL/kg) IA, blood was drawn 1hr, 2hr, 3hr, and 24hr after IA injection. Ten mice were injected with vehicle (n=10) and were served as controls. Then, the aPTT of each group was measured. The results are shown in Table 1-1 and Fig. 2A. Fig. 2A is a diagram of Table 1-1.

**Table 1-1: aPTT of IA-treated mice under different treatment times**

| Treatment time (hr) | 0 | 1 | 2 | 3 | 24 |
|---|---|---|---|---|---|
| N | 10 | 6 | 6 | 6 | 6 |
| Mean of aPTT (sec) | 26.3 | 30.8 | 32.0 | 33.7 | 34.8 |
| S.D. | 3.6 | 1.5 | 3.5 | 2.1 | 4.1 |
| *p-value | --- | 0.004 | 0.008 | <0.001 | 0.001 |

| | | | | | |
|---|---|---|---|---|---|
| *Data were compared with untreated group (treatment time is 0 hr). S.D.=standard deviation. | | | | | |

As shown in Table 1-1 and Fig. 2A, the aPTT prolonged in 1h after IA injection and was gradually prolonged as the treatment time increases. The data obtained after treating IA for 1hr have statistical meaning. Compared to PT (as shown below), aPTT was more sensitive to IA injection. The p value for trend in aPTT was <0.001. The results reveal that the coagulation time in the IA-treated mice increases with time, indicating a trend of anticoagulation in the intrinsic pathway caused by IA.

### Example 2: Dose effects (aPTT)

To investigate the anticoagulation effect in the intrinsic pathway of coagulation system in the IA-treated mice, the aPTT was also measured under different doses of IA. Various doses of IA (1 ppm, 10 ppm, 100 ppm, 1,000 ppm) in 1 mL were intraperitoneally injected into different groups of mice (n=6), respectively. Plasma was separated 3hr later. Ten mice were injected with vehicle and were served as controls. The aPTT of each group was measured. The results are shown in Table 1-2 and Fig. 2B. Fig. 2B is a diagram of Table 1-2.

**Table 1-2: aPTT of IA-treated mice with different doses**

| Injected dose (ppm) | 0 | 1 | 10 | 100 | 1000 |
|---|---|---|---|---|---|
| N | 10 | 6 | 6 | 6 | 6 |
| Mean of aPTT (sec) | 26.3 | 33.0 | 33.0 | 33.4 | 33.7 |
| S.D. | 3.6 | 4.4 | 4.5 | 2.3 | 2.1 |
| *p-value | --- | 0.005 | 0.006 | <0.001 | 0.001 |

| | | | | | |
|---|---|---|---|---|---|
| Sample were obtained 3hr after IA intraperitoneal injection *Data were compared with untreated group (injected dose is 0 ppm). S.D.=standard deviation. | | | | | |

As shown in Table 1-2 and Fig. 2B, the aPTT was prolonged when mice were treated with 1, 10, 100, 1000 ppm of IA. The data have statistical meaning when IA was treated with 1 ppm (5x10⁻⁵ mL/kg) or more. The p value for trend was 0.005. The results reveal that IA effectively prolongs the coagulation time in the IA-treated mice, indicating an anticoagulation effect in the intrinsic pathway caused by IA as low as 1 ppm.

### Example 3: Time course study (PT)

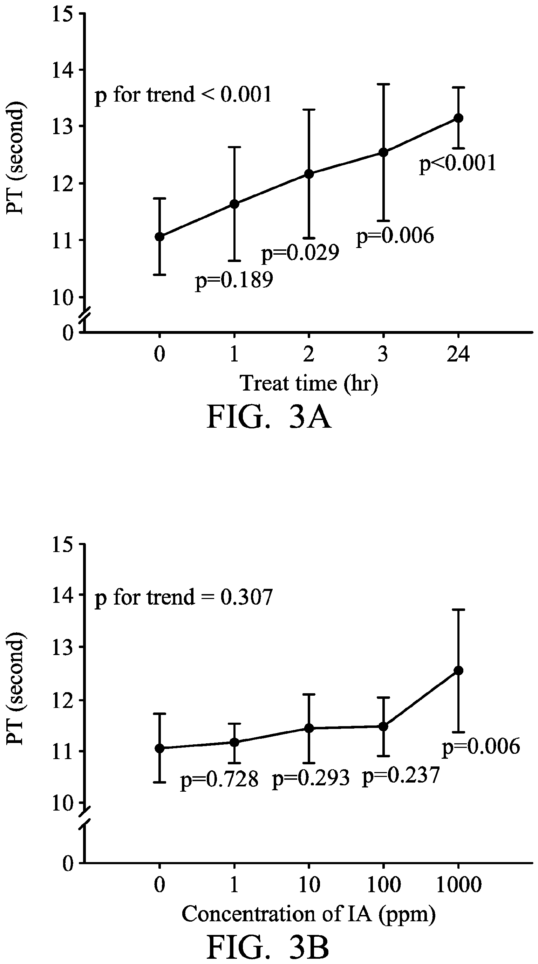

Moreover, to investigate the anticoagulation effect in the extrinsic pathway of coagulation system in the IA-treated mice, the PT was measured at different treatment times. Different groups of mice (n=6) were respectively intraperitoneally injected with 1 mL of 1000 ppm (0.05 mL/kg) IA, blood was drawn 1hr, 2hr, 3hr, and 24hr after IA injection. Ten mice were injected with vehicle (n=10) and were served as controls. Then, the PT of each group was measured. The results are shown in Table 2-1 and Fig. 3A. Fig. 3A is a diagram of Table 2-1.

**Table 2-1: PT of IA-treated mice under different treatment times**

| Treatment time (hr) | 0 | 1 | 2 | 3 | 24 |
|---|---|---|---|---|---|
| N | 10 | 6 | 6 | 6 | 6 |
| Mean of PT (sec) | 11.1 | 11.6 | 12.2 | 12.6 | 13.2 |
| S.D. | 0.7 | 1.0 | 1.1 | 1.2 | 0.5 |
| *p-value | --- | 0.189 | 0.029 | 0.006 | <0.001 |

| | | | | | |
|---|---|---|---|---|---|
| *Data were compared with untreated group (treatment time is 0 hr). S.D.=standard deviation. | | | | | |

As shown in Table 2-1 and Fig. 3A, the PT prolonged at 2h and was gradually prolonged as the treatment time increased. The data obtained after treating IA for 2hr have statistical meaning. The p value for trend in PT was <0.001. The results reveal that the coagulation time in the IA-treated mice increases with time, indicating a trend of anticoagulation in the extrinsic pathway caused by IA.

### Example 4: Dose effects (PT)

To investigate the anticoagulation effect in the extrinsic pathway of coagulation system in the IA-treated mice, the PT was also measured under different doses of IA. Various doses of IA (1 ppm, 10 ppm, 100 ppm, 1,000 ppm) in 1 mL were intraperitoneally injected into different groups of mice (n=6), respectively. Plasma was separated 3hr later. Ten mice were injected with vehicle and were served as controls. The PT of each group was measured. The results are shown in Table 2-2 and Fig. 3B. Fig. 3B is a diagram of Table 2-2.

**Table 2-2: PT of IA-treated mice with different doses**

| Injected dose (ppm) | 0 | 1 | 10 | 100 | 1000 |
|---|---|---|---|---|---|
| N | 10 | 6 | 6 | 6 | 6 |
| Mean of PT (sec) | 11.1 | 11.2 | 11.4 | 11.5 | 12.6 |
| S.D. | 0.7 | 0.4 | 0.7 | 0.6 | 1.2 |
| *p-value | --- | 0.728 | 0.293 | 0.237 | 0.006 |

| | | | | | |
|---|---|---|---|---|---|
| Sample were obtained 3hr after IA intraperitoneal injection *Data were compared with untreated group (injected dose is 0 ppm). S.D.=standard deviation. | | | | | |

As shown in Table 2-2 and Fig. 3B, the PT was obviously prolonged when mice were treated with 1000 ppm of IA. The data have statistical meaning when treating 1000 ppm of IA. The p value for trend in PT was 0.307. The results reveal that the coagulation time in the IA-treated mice increases at a high dose of IA, indicating an anticoagulation effect in the extrinsic pathway caused by a high dose of IA.

The present application shows that IA suppresses murine coagulation system, including intrinsic pathway (aPTT) and extrinsic pathway (PT). The PT was suppressed by 1 mL of 1000 ppm IA (0.05 mL/kg) 2h after intraperitoneal injection. However, in the dose study, it needed 1 mL of 1000 ppm IA to suppress PT 3hr after intraperitoneal injection. aPTT is more sensitive to IA suppression. IA injection with 1 mL of 1000 ppm was able to suppress aPTT in 1 hr. For dose study, as low as 1 ppm of 1 mL IA was able to suppress aPTT 3hr after IA intraperitoneal injection. The above data show that IA effectively prolongs aPTT of the-treated mice, but prolongs the PT only at high dose of IA such as 1000 ppm, which suggests that IA has more significant effect on the intrinsic pathway of coagulation than that of the extrinsic pathway. In other words, the intrinsic pathway is more sensitive to IA suppression.

It should be noted that, our experimental data also show that a normal physiological state can be maintained when 1 mL of 1,000 ppm (0.05 mL/kg) IA was applied to mice. BALB/C mice (20g) could tolerate 1 mL of 20,000 ppm (0.1 mL/kg) of intraperitoneal injection. Moreover, Applicant also found that a normal physiological state can also be maintained even when 1mL of 30,000 ppm IA was applied to rats. In other words, IA is not harmful to the health of mammals even at high doses.

The results of the present invention illustrate that both of the PT of extrinsic pathway and the aPTT of intrinsic pathway are prolonged as the treatment time of IA increased. Also, it was found that PT increased with statistical meaning after 2 hr of the intraperitoneal injection of 1mL of 1000 ppm IA and the aPTT increased with statistical meaning after 1 hr of the intraperitoneal injection of 1mL of 1000 ppm IA.

These results reveal that the pharmaceutical composition of formula (I), i.e. IA, has an anticoagulation effect in mammals such as mice and rats. Therefore, the method for anticoagulation by administering IA to a subject in need has a very high value in future medical research and application. For example, the use of administering IA for anticoagulation in prevention of thrombosis and embolism.

While the invention has been described by way of example and in terms of the preferred embodiments, it should be understood that the invention is not limited to the disclosed embodiments. On the contrary, it is intended to cover various modifications and similar arrangements (as would be apparent to those skilled in the art). Therefore, the scope of the appended claims should be accorded the broadest interpretation so as to encompass all such modifications and similar arrangements.

## Claims

1. A pharmaceutical composition for use in anticoagulation, wherein the pharmaceutical composition comprises formula (I): 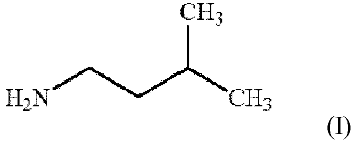 or its isomers as its parent form, and its salt, ester or solvate.

2. The pharmaceutical composition as claimed in claim 1, wherein the isomers comprise formulas (II), (III) or (IV): 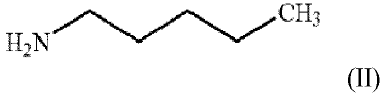 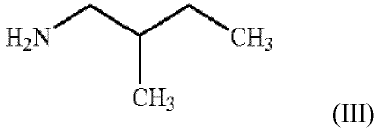 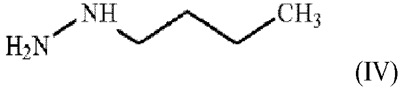 and their salt, ester or solvate.

3. The pharmaceutical composition as claimed in claim 1 or 2, wherein the pharmaceutical composition further comprises a pharmaceutically acceptable carrier, wherein the pharmaceutically acceptable carrier is phosphate buffered saline.

4. The pharmaceutical composition as claimed in any one of claims 1-3, wherein the pharmaceutical composition is administered parenterally.

5. The pharmaceutical composition as claimed in any one of claims 1-3, wherein the pharmaceutical composition is administered by intraperitoneal injection or intravascular injection.

6. The pharmaceutical composition as claimed in any one of claims 1-3, wherein the pharmaceutical composition is administered orally in a liquid dosage form or in a solid dosage form.

7. The pharmaceutical composition as claimed in any one of claims 1-6, wherein the subject is a mammal, wherein the mammal is a mouse or a human.

8. A pharmaceutical composition for use in anticoagulation, wherein the pharmaceutical composition comprises formula (I): 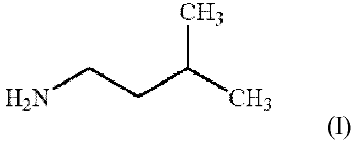 or its isomers as its parent form, and its salt, ester or solvate, wherein a dose of the pharmaceutical composition for use in anticoagulation is in an amount of about 5x10⁻⁵ mL/kg or more of body weight of the subject.

9. The pharmaceutical composition as claimed in claim 8, wherein the isomers comprise formulas (II), (III) or (IV): 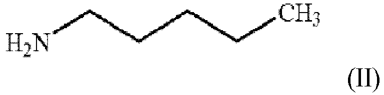 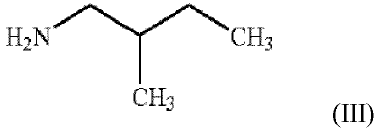 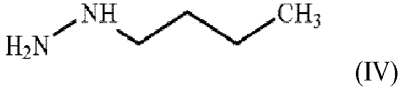 and their salt, ester or solvate.

10. The pharmaceutical composition as claimed in claim 8 or 9, wherein the dose of the pharmaceutical composition for use in anticoagulation is in an amount of 0.05 mL/kg of body weight of the subject.

11. The pharmaceutical composition as claimed in claim 8 or 9, wherein the dose of the pharmaceutical composition for use in anticoagulation is in an amount of 5x10⁻⁵ mL/kg of body weight of the subject.

12. The pharmaceutical composition as claimed in any one of claims 8-11, wherein the pharmaceutical composition further comprises a pharmaceutically acceptable carrier, wherein the pharmaceutically acceptable carrier is phosphate buffered saline.

13. The pharmaceutical composition as claimed in any one of claims 8-12, wherein the pharmaceutical composition is administered parenterally.

14. The pharmaceutical composition as claimed in any one of claims 8-12, wherein the pharmaceutical composition is administered orally in a liquid dosage form or in a solid dosage form.

15. The pharmaceutical composition as claimed in any one of claims 8-14, wherein the subject is a mammal, wherein the mammal is a mouse or a human.
